# EUROPEAN PATENT SPECIFICATION

(11) **EP 2 189 812 B1**
(45) Date of publication and mention of the grant of the patent: **11.05.2016**
(21) Application number: 09175747.6
(22) Date of filing: 12.11.2009
(51) Int. Cl.: G01S 15/89, G01S 7/52, A61B 8/08, G06T 7/60

(54) **Providing volume information on a periodically moving target object in an ultrasound system**
Bereitstellung der Volumeninformation eines sich regelmäßig bewegenden Zielobjekts in einem Ultraschallsystem
Fourniture d'informations sur le volume sur un objet cible en mouvement périodique dans un système d'ultrasons

(30) Priority: 25.11.2008 KR 20080117313; 02.11.2009 KR 20090104738
(43) Date of publication of application: 26.05.2010
(73) Proprietor: Samsung Medison Co., Ltd., Gangwon-do (KR)
(72) Inventor: Yoo, Jae Heung, 135-851 Seoul (KR)
(74) Representative: Schmid, Wolfgang

(56) References cited:
- US-A1- 2006 074 315
- NADKARNI S K ET AL: "Image-based retrospective cardiac gating for three-dimensional intravascular ultrasound imaging" PROCEEDINGS OF THE SPIE - THE INTERNATIONAL SOCIETY FOR OPTICAL ENGINEERING, SPIE, US, vol. 4687, 1 January 2002 (2002-01-01), pages 276-284, XP002272220 ISSN: 0277-786X
- KEREM KARADAYI ET AL: "Automatic Image-based Gating for 4D Ultrasound" ENGINEERING IN MEDICINE AND BIOLOGY SOCIETY, 2006. EMBS '06. 28TH ANNUAL INTERNATIONAL CONFERENCE OF THE IEEE, IEEE, PISCATAWAY, NJ, USA, 30 August 2006 (2006-08-30), pages 2388-2391, XP031235999 ISBN: 978-1-4244-0032-4
- NADKARNI S K ET AL: "Image-based cardiac gating for three-dimensional intravascular ultrasound imaging" ULTRASOUND IN MEDICINE AND BIOLOGY, NEW YORK, NY, US, vol. 31, no. 1, 1 January 2005 (2005-01-01), pages 53-63, XP004711525 ISSN: 0301-5629
- BERG S ET AL: "Dynamic three-dimensional freehand echocardiography using raw digital ultrasound data" ULTRASOUND IN MEDICINE AND BIOLOGY, NEW YORK, NY, US, vol. 25, no. 5, 1 June 1999 (1999-06-01), pages 745-753, XP004295398 ISSN: 0301-5629

## Description

### TECHNICAL FIELD

The present disclosure relates to ultrasound systems, and more particularly to providing volume information regarding a periodically moving target object in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

Recently, the ultrasound system has been improved to provide a three-dimensional ultrasound image. A static three-dimensional ultrasound image is often used for ultrasound diagnostic purposes. By using the static three-dimensional ultrasound image, it is possible to perform accurate observations, diagnoses or treatments of a human body without conducting complicated procedures such as invasive operations. However, the static three-dimensional image may not be useful in certain cases, for example, in observing a moving target object such as a heart, a fetus in the uterus or the like in real time.

Further, there has been an increased interest in heart conditions of a fetus since there is an increasing need to perform an early diagnosis of the fetus' status. However, it is impossible to measure exact volume of the moving heart.

According to XP 002272220 "Image Based Retrospective Cardiac Gating for Three-Dimensional Intravascular Ultrasound Imaging" a tracing direction is determined by û1, which is the unit vector describing the direction from the location of the previous trace point pᵢ₋₁ to the location of the current trace point pᵢ. At the current tracing point pᵢ, the next tracing point pᵢ₊₁ is searched by stepping a distance equal to a user defined step size in the direction of the unit vector û₁. The next tracing point pᵢ₊₁ is detected by an iterative process, from a set of possible candidate trace points selected along a window. The determination of the each tracing point is based on fining the minimum of cost function computed at each of the candidate trace points in the window and the location of the next optimal trace point pᵢ₊₁, from the set of candidate trace points is determined at the local minimum of the net energy function.

XP 031235999 "Automatic Image-based Gating for 4D Ultrasound" by Kerem Karadayi et. al. discloses a new image gating technique having low computational requirements and being fully automatic. This document states that centroidₓ(t) and centroidy(t) signals are computed and the a periodogram is computed to identify the dominant frequency.

A medical diagnostic ultrasound characterization of cardiac motion is known from US 2006/0074315 A1. According to the method described therein, a sequence of images is associated with one or more cycle, a common portion, such as a systole or diastole portion, is extracted. A cardiac cycle parameter is identified as a function of a change in the cavity area and once the cardiac cycle parameter is identified, frames of data associated with a desired time or time period are extracted.

### SUMMARY

Embodiments for forming an elastic image in an ultrasound system are disclosed herein. According to the present invention, there is provided an ultrasound system according to claim 1.

Furthermore, according to the invention method of providing volume information of a periodically moving target object according to claim 9 is provided.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit.
- FIG. 3: is a block diagram showing an illustrative embodiment of a processing unit.
- FIG. 4: is a block diagram showing an illustrative embodiment of a period detecting section.
- FIG. 5: is a flowchart showing an illustrative embodiment of forming volume information of a periodically moving target object.
- FIG. 6: is a schematic diagram showing an example of acquiring ultrasound data corresponding to a plurality of frames.
- FIG. 7: is a schematic diagram showing an example of setting a feature point at each of the slice images.
- FIG. 8: is a schematic diagram showing an example of forming a feature point curve based on distances between a principal axis and feature points.
- FIG. 9: is a schematic diagram showing an example of a feature point curve.
- FIG. 10: is a schematic diagram showing a procedure of reconstructing volume data based on a moving period of a target object.

### DETAILED DESCRIPTION

A detailed description may be provided with reference to the accompanying drawings. One of ordinary skill in the art may realize that the following description is illustrative only and is not in any way limiting.

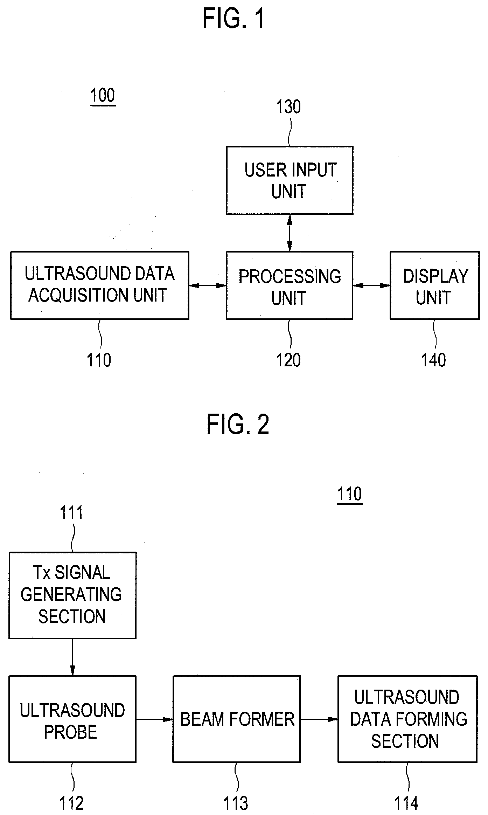

Referring to FIG. 1, an ultrasound system 100 in accordance with an illustrative embodiment is shown. As depicted therein, the ultrasound system 100 includes an ultrasound data acquisition unit 110. The ultrasound data acquisition unit 110 is operable to transmit/receive ultrasound signals to/from a periodically moving target object to thereby output ultrasound data. The ultrasound data acquisition unit 110 includes a transmit (Tx) signal generating section 111, as shown in FIG. 2.

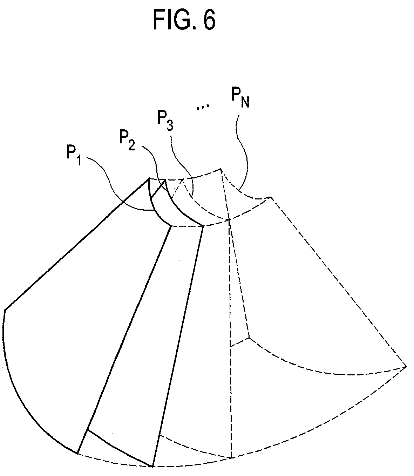

The Tx signal generating section 111 is operable to generate Tx signals. The Tx signal generating section 111 performs the generation of the Tx signals at every predetermined time to thereby form a plurality of Tx signals for obtaining each of frames Pᵢ(1≤i≤N) representing the target object, as shown in FIG. 6. The frame represents sectional planes of the target object.

The ultrasound data acquisition unit 110 further includes an ultrasound probe 112 containing a plurality of elements for reciprocally converting ultrasound signals and electrical signals. The ultrasound probe 112 is operable to transmit ultrasound signals into the target object in response to the Tx signals. The ultrasound probe 112 is further operable to receive echo signals reflected from the target object to thereby output received signals. The received signals are analog signals. The ultrasound probe 112 includes a three-dimensional probe, a two-dimensional array probe or the like.

The ultrasound data acquisition unit 110 further includes a beam former 113. The beam former 113 is operable to convert the received signals into digital signals. The beam former 113 is further operable to apply delays to the digital signals in consideration of distances between the elements and focal points to thereby output digital receive-focused signals.

The ultrasound data acquisition unit 110 further includes an ultrasound data forming section 114. The ultrasound data forming section 114 is operable to form ultrasound data based on the digital receive-focused signals.

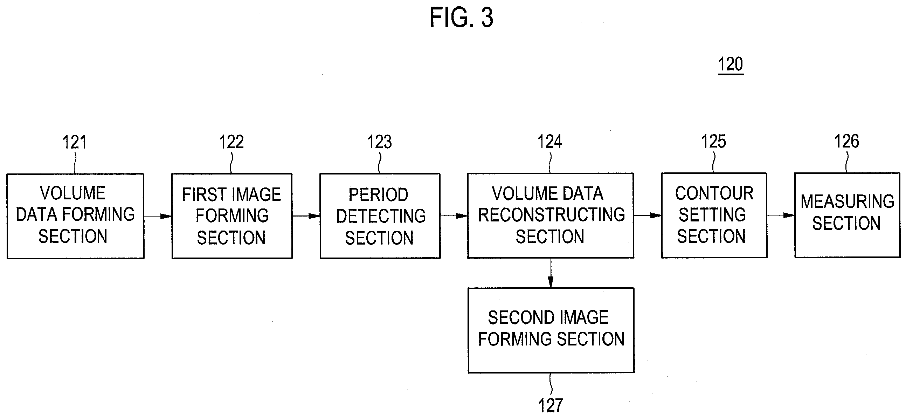

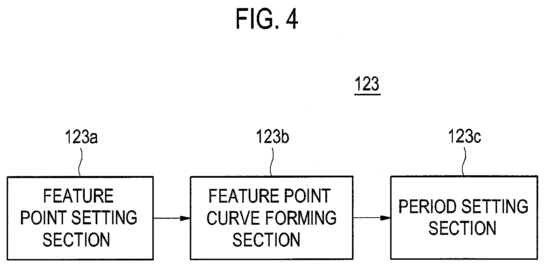

Referring back to FIG. 1, the ultrasound system 100 further includes a processing unit 120, which is coupled to the ultrasound data acquisition unit 110. Referring to FIG. 3, the processing unit 120 includes a volume data forming section 121, a first image forming section 122, a period detecting section 123, a volume data reconstruction section 124, a contour setting section 125, a measuring section 126 and a second image forming section 127. Also, the period detecting section 123 includes a feature point setting section 123a, a feature point curve forming section 123b and a period setting section 123c, as shown in FIG. 4.

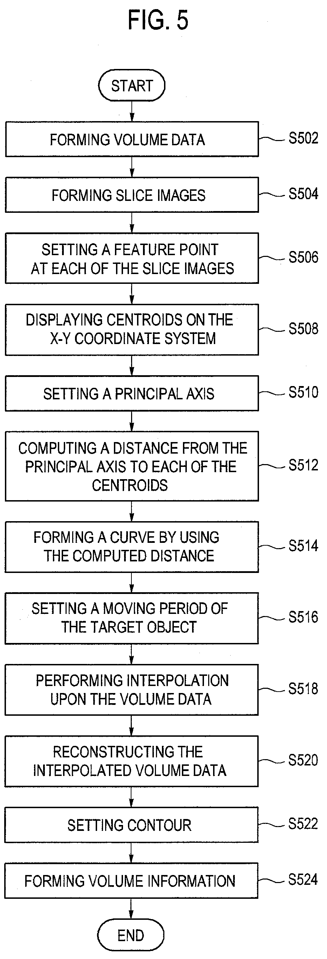

FIG. 5 is a flowchart showing an illustrative embodiment of forming volume information of a periodically moving target object. Referring to FIG. 5, it will be explained as to how the volume information is formed with respect to the periodically moving target object.

The volume data forming section 121 is operable to synthesize the ultrasound data corresponding to the frames Pᵢ(1≤i≤N) to thereby form volume data including the frames Pᵢ(1≤i≤N), as shown in FIG. 5 (S502).

The first image forming section 122 is operable to form the plurality of slice images based on the volume data (S504). The slice image is a brightness mode image corresponding to the frame. Also, the slice image includes pixels each having a brightness value.

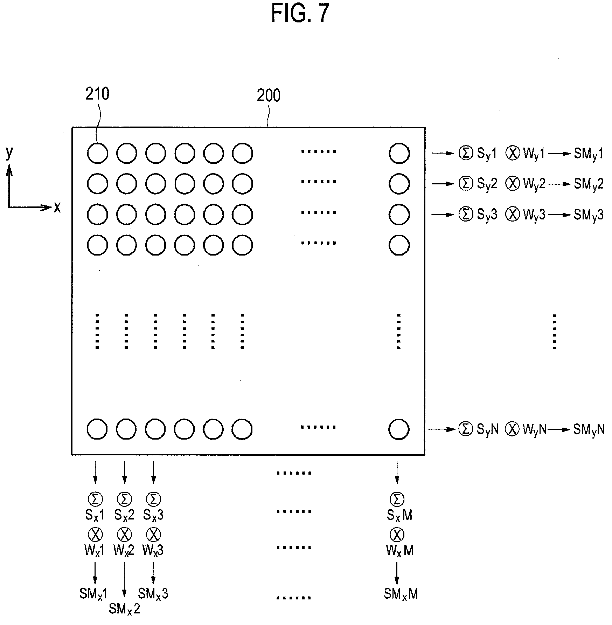

The feature point setting section 123a is operable to set a feature point on each of the slice images formed by the first image forming section 122 (S506). The feature point is set by using common feature on each of the slice images. In one embodiment, the feature point is set by using a centroid of brightness values constituting each of the slice images. A method of determining a centroid of brightness values will be described by using a slice image 200 having M × N pixels 210, as shown in FIG. 7 as an example.

FIG. 7 is a schematic diagram showing an example of setting the feature point at each of the slice images. For the sake of convenience, it will be described that the slice images are placed on the X-Y plane of rectangular coordinate system in which the X coordinates of the slice image range from 1 to M and the Y coordinates of the slice image range from 1 to N. The feature point setting section 123a is operable to vertically sum the pixel values at each of the X coordinates 1 to M in the slice image. That is, assuming that brightness values in the slice image are represented by P_{XY}, the feature point setting section 123a is operable to sum P_{X1}, P_{X2}, ...and P_{XN} to thereby output first sums Sx1-SxM corresponding to the respective X coordinates. Subsequently, the feature point setting section 123a further multiplies the first sums Sx1-SxM by weights Wx1-WxM, respectively, to thereby output first weighted sums SMx1-SMxM. In one embodiment, the weights Wx1-WxM is determined by arbitrary values, which increase or decrease at a constant interval. For example, the numbers 1-M are used as the weight values Wx1-WxM. The feature point setting section 123a is further operable to sum all of the first sums Sx1-SxM to thereby output a second sum, as well as sum all of the first weighted sums SMx1-SMxM to thereby output a third sum. The feature point setting section 123a further divides the third sum by the second sum. Then it sets the division result as the centroid on the X axis.

Also, the feature point setting section 123a is operable to horizontally sum the pixel values at each of the Y coordinates 1-N in the slice image. That is, assuming that brightness values in the slice image are represented by P_{XY}, the feature point setting section 123a is operable to sum P_{1Y}, P_{2Y}, ... and P_{MY} to thereby output fourth sums Sy1-SyN corresponding to the respective Y coordinates. Subsequently, the feature point setting section 123a further multiplies the fourth sums Sy1-SyN by weights Wy1-WyN, respectively, to thereby output second weighted sums SMy1-SMyN. In one embodiment, the weights Wy1-WyN are determined by arbitrary values, which increase or decrease at a constant interval. For example, the numbers 1-N are used as the weight values Wy1-WyN. The feature point setting section 123a is further operable to sum all of the fourth sums Sy1-SyN to thereby output a fifth sum, as well as sum all of the second weighted sums SMy1-SMyN to thereby output a sixth sum. The feature point setting section 123a further divides the sixth sum by the fifth sum and then set the division result as the centroid on the Y axis.

Although it is described that the feature point is set by using the centroid of brightness values constituting each of the slice images, the feature point setting is certainly not limited thereto. The feature point at each of the slice images is set through singular value decomposition upon each of the slice images.

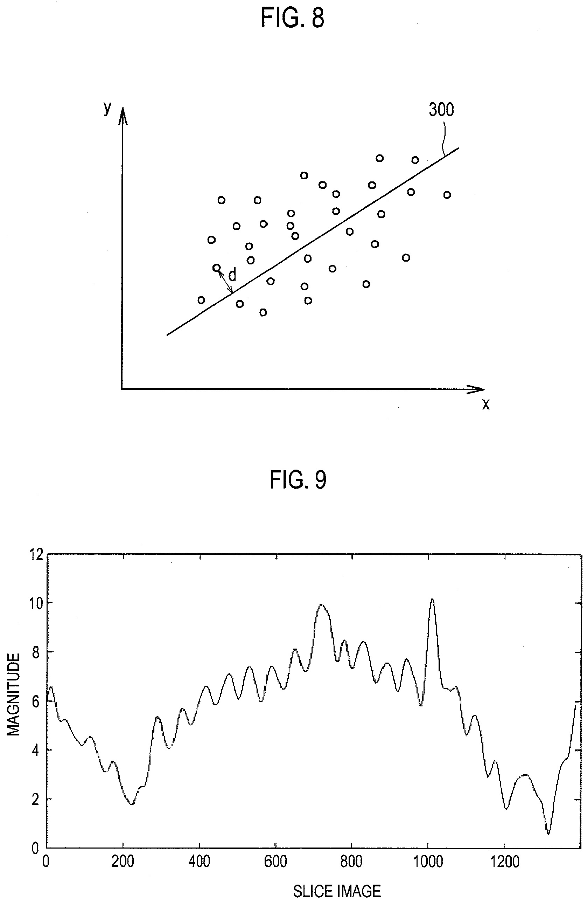

Once the setting of the centroid is complete for all of the slice images, the feature point curve forming section 123b displays centroids on the X-Y coordinate system (S508) and then set a principal axis 300 thereon (S510), as illustrated in FIG. 8.

FIG. 8 is a schematic diagram showing an example of forming the feature point curve based on distances between the principal axis and feature points. The feature point curve forming section 123b is further operable to compute distances "d" from the principal axis 300 to each of the centroids (S512). The feature point curve forming section 123b further forms a curve by using the computed distances (S514), as illustrated in FIG. 9.

FIG. 9 is a schematic diagram showing an example of the feature point curve. The period setting section 123c is operable to set a moving period of the target object by using peak points in the graph illustrated in FIG. 9 (S516). In one embodiment, the period setting section 123c is operable to calculate the gradients in the curve in FIG. 9. The period setting section 123c further calculates zero cross points, the gradient of which changes from positive to negative, and then detect the zero cross points having a similar distance, thereby setting a period of the detected zero cross points to the moving period of the target object.

Referring back to FIG. 5, the volume data reconstructing section 124 is operable to interpolate the volume data to have the same number of the frames within each of the periods (S518). After completing the interpolation, the volume data reconstructing section 124 further reconstructs the interpolated volume data (S520).

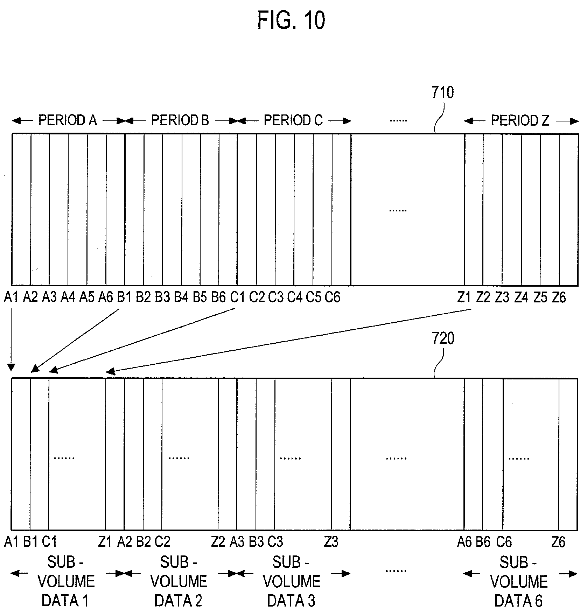

FIG. 10 shows a procedure of reconstructing the interpolated volume data. As shown in FIG. 10, twenty-six local periods A to Z exist in one volume data 710. Assuming that six frames are contained in each of periods in the interpolated volume data as shown in FIG. 10, the reconstructed volume data 720 includes six sub-volume data. Each of the sub-volume data includes 26 frames Aᵢ to Zᵢ.

Further, when the volume data are acquired by scanning the target object, the object (e.g., expectant mother or fetus) is moved. This makes it difficult to accurately detect the heartbeat of the fetus. Accordingly, the volume data reconstructing section 124 is further operable to compensate the motion of the expectant mother or the fetus by matching the brightness of pixels. The detailed description relating to the method of compensating the motion is omitted herein since the conventional methods are used.

The contour setting section 125 is operable to set contour of the target object on each of the sub-volume data (S522). In one embodiment, the contour setting section 125 is operable to set the contour on each of the sub-volume data based on a user instruction from a user input unit 130. In another embodiment, the contour setting section 125 automatically detects contour points at each of the sub-volume data to thereby set the contour on each of the sub-volume data based on the detected contour points. The detailed description relating to the method of detecting the contour points is omitted herein since the conventional methods are used.

The measuring section 126 measures the volume of the target object based on the contour of the target object set by the contour setting section 125 to thereby form volume information (S524). The detailed description relating to the method of measuring the volume with the contour is omitted herein since the conventional methods are used.
Referring back to FIG. 3, the second image forming section 127 is operable to render each of the sub-volume data to thereby form three-dimensional ultrasound images. A user inputs a user instruction to set a contour of the target object by using three-dimensional ultrasound images.
Referring again to FIG. 1, the ultrasound system 100 further includes the user input unit 130. The user input unit 130 is operable to receive the user instruction from a user. The user instruction includes an instruction to set the contour of the target object on each of the three-dimensional ultrasound images. The user input unit 130 includes a control panel (not shown), a mouse (not shown), a keyboard (not shown) or the like.
The ultrasound system 100 further includes a display unit 140. The display unit 140 displays the volume information from the processing unit 120. The display unit 140 further displays the three-dimensional ultrasound images from the processing unit 120.

## Claims

1. An ultrasound system (100), comprising:
an ultrasound data acquisition unit (110) configured to transmit/receive ultrasound signals from/to a periodically moving target object to thereby form ultrasound data; and
a processing unit (120) coupled to the ultrasound data acquisition unit (110), wherein the processing unit (120) comprises:
a volume data forming section (121) configured to form volume data including a plurality of frames based on the ultrasound data;
a first image forming section (122) configured to form a plurality of slice images based on the volume data;
a period detecting section (123) configured to set a feature point on each of the slice images based on brightness values of pixels included therein and form a feature point curve based on the feature points, the period detecting section (123) being further configured to calculate zero crossing points in the gradient of the feature point curve and detect a period of the detected zero crossing points as a moving period of the target object, wherein the moving period comprises a plurality of local periods;
a volume data reconstructing section (124) configured to interpolate the volume data to have the same number of the frames within each local period and reconstruct the interpolated volume data into a plurality of sub-volume data, each sub-volume data is constructed by using frames at an identical position in each local period;
a contour setting section (125) configured to set contour of the target object on each of the sub-volume data; and
a measuring section (126) configured to measure the volume of the target object based on the contour to thereby form the volume information.

2. The ultrasound system (100) of Claim 1, wherein the feature point is a centroid of the brightness values on each of the slice images.

3. The ultrasound system (100) of Claim 1, wherein the period detecting section (123) is configured to set a principal axis based on positions of the feature points and form the feature point curve based on distances between the feature points and the principal axis.

4. The ultrasound system (100) of Claim 1, wherein the period detecting section (123) is configured to calculate the gradients from the feature point curve, detect zero crossing points that a sign of the gradient changes from positive to negative and determine the moving period based on intervals between the detected zero crossing points.

5. The ultrasound system (100) of Claim 1, wherein the processing unit (120) further comprises a second image forming section (127) configured to render the plurality of sub-volume data to thereby form three-dimensional ultrasound images.

6. The ultrasound system (100) of Claim 5, further comprising a user input unit (130) configured to receive a user instruction for setting the contour of the target object on each of the three-dimensional ultrasound images.

7. The ultrasound system (100) of Claim 6, wherein the contour setting section (125) is configured to set the contour of the target object on each of the sub-volume data based on the user instruction.

8. The ultrasound system (100) of Claim 1, wherein the contour setting section (125) is configured to detect contour points at each of the sub-volume data and set the contour on each of the sub-volume data based on the detected contour points.

9. A method of providing volume information of a periodically moving target object, comprising:
a) transmitting/receiving ultrasound signals from/to a periodically moving target object to thereby form ultrasound data;
b) forming volume data including a plurality of frames based on the ultrasound data;
c) forming a plurality of slice images based on the volume data;
d) setting a feature point on each of the slice images based on brightness values of pixels included therein to form a feature point curve based on the feature points;
e) calculating zero crossing points in the gradient of the feature point curve to detect a period of then detected zero crossing points as a moving period of the target object, wherein the moving period comprises a plurality of local periods;
f) interpolating the volume data to have the same number of the frames within each local period;
g) reconstructing the interpolated volume data into a plurality of sub-volume data, each sub-volume data is constructed by using frames at an identical position in each local period;
h) setting contour of the target object on each of the sub-volume data; and
i) measuring the volume of the target object based on the contour to thereby form volume information.

10. The method of Claim 9, wherein the step d) comprises:
setting the feature point on each of the slice images based on a centroid of the brightness values at each of the slice images;
setting a principal axis based on positions of the feature points; and
forming the feature point curve based on distances between the feature points and the principal axis.

11. The method of Claim 9, wherein the step e) comprises:
calculating the gradients from the feature point curve;
detecting zero crossing points that a sign of the gradient changes from positive to negative; and
determining the moving period based on intervals between the detected zero crossing points.

12. The method of Claim 9, wherein the step h) comprises:
setting the contour of the target object on each of the sub-volume data based on a user instruction.

13. The method of Claim 9, wherein the step h) comprises:
detecting contour points of the target object at each of the sub-volume data; and
setting the contour on each of the sub-volume data based on the detected contour points.

## Patentansprüche

1. Ultraschallsystem (100), welches Folgendes aufweist:
eine Ultraschalldatenerlangungseinheit (110), die dafür vorgesehen ist, Ultraschallsignale zu bzw. von einem sich periodisch bewegenden Zielobjekt zu erzeugen bzw. zu empfangen, um dadurch Ultraschalldaten zu bilden; und
eine Verarbeitungseinheit (120), die mit der Ultraschalldatenerlangungseinheit (110) gekoppelt ist,
wobei die Verarbeitungseinheit (120) Folgendes aufweist:
einen Volumendatenerzeugungsabschnitt (121), der dafür vorgesehen ist, eine Vielzahl von Rahmen aufweisende Volumendaten basierend auf den Ultraschalldaten zu erzeugen;
einen ersten Bilderzeugungsabschnitt (122), der dafür vorgesehen ist, eine Vielzahl von Scheiben- bzw. Teilbildern, basierend auf den Volumendaten zu erzeugen;
einen Periodenermittlungsabschnitt (123), der dafür vorgesehen ist, einen Merkmalspunkt auf jedem der Teilbilder, basierend auf Helligkeitswerten von darin enthaltenen Pixeln festzulegen und eine Merkmalspunktkurve basierend auf den Merkmalspunkten zu erzeugen, wobei der Periodenermittlungsabschnitt (123) des Weiteren dafür vorgesehen ist, Nulldurchgangs- bzw. Nullkreuzungspunkte in dem Gradienten der Merkmalspunktkurve zu berechnen und eine Periode der detektierten Nullkreuzungspunkte als eine sich bewegende Periode des Zielobjekts zu detektieren, wobei die sich bewegende Periode eine Vielzahl von lokalen Perioden aufweist;
einen Volumendatenrekonstruktionsabschnitt (124), der dafür vorgesehen ist, die Volumendaten derart zu interpolieren, dass sie dieselbe Anzahl von Rahmen innerhalb jeder lokalen Periode aufweisen, und die interpolierten Volumendaten in eine Vielzahl von Untervolumendaten zu rekonstruieren, wobei jede der Untervolumendaten unter Verwendung von Rahmen an einer identischen Position in jeder lokalen Periode konstruiert wird;
einen Konturfestlegungsabschnitt (125), der dafür vorgesehen ist, die Kontur des Zielobjekts auf jeder der Untervolumendaten festzulegen; und
einen Messabschnitt (126), der dafür vorgesehen ist, das Volumen des Zielobjekts basierend auf der Kontur zu messen, um dadurch die Volumeninformationen zu erzeugen.

2. Ultraschallsystem (100) nach Anspruch 1, wobei der Merkmalspunkt ein Schwerpunkt der Helligkeitswerte auf jedem der Teilbilder ist.

3. Ultraschallsystem (100) nach Anspruch 1, wobei der Periodenermittlungsabschnitt (123) dafür vorgesehen ist, eine Hauptachse basierend auf Positionen der Merkmalspunkte festzulegen und die Merkmalspunktkurve basierend auf Abständen zwischen den Merkmalspunkten und der Hauptachse zu erzeugen.

4. Ultraschallsystem (100) nach Anspruch 1, wobei der Periodenermittlungsabschnitt (123) dafür vorgesehen ist, die Gradienten aus der Merkmalspunktkurve zu berechnen, Nullkreuzungspunkte zu detektieren, bei denen ein Zeichen des Gradienten sich von positiv zu negativ ändert, und die sich bewegende Periode basierend auf Intervallen zwischen den detektieren Nullkreuzungspunkten zu ermitteln.

5. Ultraschallsystem (100) nach Anspruch 1, wobei die Verarbeitungseinheit (120) des Weiteren einen zweiten Bilderzeugungsabschnitt (127) aufweist, der dafür vorgesehen ist, die Vielzahl von Untervolumendaten zu rendern, um dadurch dreidimensionale Ultraschallbilder zu erzeugen.

6. Ultraschallsystem (100) nach Anspruch 5, welches des Weiteren eine Benutzereingabeeinheit (130) aufweist, die dafür vorgesehen ist, einen Benutzerbefehl zum Festlegen der Kontur des Zielobjekts auf jedem der dreidimensionalen Ultraschallbilder zu empfangen.

7. Ultraschallsystem (100) nach Anspruch 6, wobei der Konturfestlegungsabschnitt (125) dafür vorgesehen ist, die Kontur des Zielobjekts auf jeder der Untervolumendaten basierend auf dem Benutzerbefehl festzulegen.

8. Ultraschallsystem (100) nach Anspruch 1, wobei der Konturfestlegungsabschnitt (125) dafür vorgesehen ist, Konturpunkte an jedem der Untervolumendaten zu detektieren und die Kontur auf jeder der Untervolumendaten basierend auf den detektierten Konturpunkten festzulegen.

9. Verfahren zum Erzeugen von Volumeninformationen eines sich periodisch bewegenden Zielobjekts, welches Folgendes aufweist:
a) Übertragen bzw. Empfangen von Ultraschallsignalen zu bzw. von einem sich periodisch bewegenden Zielobjekt, um dadurch Ultraschalldaten zu erzeugen;
b) Erzeugen einer Vielzahl von Rahmen aufweisender Volumendaten basierend auf den Ultraschalldaten;
c) Erzeugen einer Vielzahl von Scheiben- bzw. Teilbildern basierend auf den Volumendaten;
d) Festlegen eines Merkmalspunkts auf jedem der Teilbilder basierend auf Helligkeitswerten von darin enthaltenen Pixeln, um eine Merkmalspunktkurve basierend auf den Merkmalspunkten zu erzeugen;
e) Berechnen von Nulldurchgangs- bzw. Nullkreuzungspunkten in dem Gradienten der Merkmalspunktkurve, um eine Periode der detektierten Nullkreuzungspunkte als eine sich bewegende Periode des Zielobjekts zu detektieren, wobei die sich bewegende Periode eine Vielzahl von lokalen Perioden aufweist,
f) Interpolieren der Volumendaten, derart, dass sie dieselbe Anzahl von Rahmen innerhalb jeder lokalen Periode aufweisen;
g) Rekonstruieren der interpolierten Volumendaten in eine Vielzahl von Untervolumendaten, wobei jede der Untervolumendaten unter Verwendung von Rahmen an einer identischen Position in jeder lokalen Periode konstruiert wird;
h) Festlegen einer Kontur des Zielobjekts auf jeder der Untervolumendaten; und
i) Messen des Volumens des Zielobjekts basierend auf der Kontur, um dadurch Volumeninformationen zu erzeugen.

10. Verfahren nach Anspruch 9, wobei der Schritt d) Folgendes aufweist:
Festlegen des Merkmalspunkts auf jedem der Teilbilder basierend auf einem Schwerpunkt der Helligkeitswerte auf jedem der Teilbilder;
Festlegen einer Hauptachse basierend auf Positionen der Merkmalspunkte; und
Erzeugen der Merkmalspunktkurve basierend auf Abständen zwischen den Merkmalspunkten und der Hauptachse.

11. Verfahren nach Anspruch 9, wobei der Schritt e) Folgendes aufweist:
Berechnen der Gradienten aus der Merkmalspunktkurve;
Detektieren der Nullkreuzungspunkte, bei denen sich ein Zeichen des Gradienten von positiv zu negativ ändert; und
Ermitteln der sich bewegenden Periode basierend auf Intervallen zwischen den detektierten Nullkreuzungspunkten.

12. Verfahren nach Anspruch 9, wobei der Schritt h) Folgendes aufweist:
Festlegen der Kontur des Zielobjekts auf jedem der Untervolumendaten basierend auf einem Benutzerbefehl.

13. Verfahren nach Anspruch 9, wobei der Schritt h) Folgendes aufweist:
Detektieren von Konturpunkten des Zielobjekts an jedem der Untervolumendaten; und
Festlegen der Kontur auf jeder der Untervolumendaten basierend auf den detektierten Konturpunkten.

## Revendications

1. Système ultrasonique (100) comportant :
une unité d'acquisition de données ultrasoniques (110) configurée pour transmettre/recevoir des signaux ultrasoniques de/vers un objet cible périodiquement mobile pour constituer par ce moyen des données ultrasoniques; et
une unité de traitement (120) couplée à l'unité d'acquisition de données ultrasoniques (110),
dans lequel l'unité de traitement (120) comprend :
une section de formation de données de volumes (121) configurée pour former des données de volume comprenant une pluralité de cadres basés sur les données ultrasoniques ;
une section de formation d'une première image (122) configurée pour former une pluralité d'images en tranches basées sur les données de volumes ;
une section de détection de période (123) configurée pour fixer un point objet sur chacune des images en tranches sur la base des valeurs de brillance des pixels qui y sont inclus, et former une courbe de points objets basée sur les points objets, la section de détection de période (123) étant en outre configurée pour calculer les points de croisement zéro dans le gradient de la courbe de points objets et détecter une période du point de croisement zéro détecté, comme étant une période mobile d'un objet cible, dans lequel la période mobile comprend une pluralité de périodes locales ;
une section de reconstruction de volume (124) configurée pour effectuer une interpolation des données de volume pour avoir le même nombre de cadres dans chacune des périodes locales et pour reconstruire les données de volume de l'interpolation dans une pluralité de données de sous-volume, chaque donnée de sous-volume étant construite en utilisant les cadres dans une position identique dans chacune des périodes locales :
une section de détermination de contour (125) configurée pour déterminer le contour de l'objet cible dans chacune des données de sous-volume ; et
une section de mesure (126) configurée pour mesurer le volume de l'objet cible basé sur le contour afin de former ainsi l'information de volume.

2. Système ultrasonique (100) selon la revendication 1, dans lequel le point objet est le centre géométrique des valeurs de brillance sur chacune des images en tranche.

3. Système ultrasonique (100) selon la revendication 1, dans lequel la section de détection de la période (123) est configurée pour déterminer un axe principal basé sur les positions des points objets et pour former la courbe des points objets basée sur la distance entre les points objets et l'axe principal.

4. Système ultrasonique (100) selon la revendication 1, dans lequel la section de détection de la période (123) est configurée pour calculer les gradients de la courbe des points objets, détecter les points de croisement zéro pour lesquels un signe du gradient change de positif en négatif et déterminer la période de déplacement basée sur les intervalles entre les points de croisement zéro détectés.

5. Système ultrasonique (100) selon la revendication 1, dans lequel l'unité de traitement (120) comporte en outre une seconde section de formation d'image (127) configurée pour découper la pluralité de données de sous-volume pour former ainsi des images ultrasoniques tridimensionnelles.

6. Système ultrasonique (100) selon la revendication 5, comportant en outre une unité d'entrée utilisateur (130) configurée pour recevoir des instructions d'utilisateur pour déterminer le contour de l'objet cible sur chacune des images ultrasoniques tridimensionnelles.

7. Système ultrasonique (100) selon la revendication 6, dans lequel la section de détermination de contour (125) est configurée pour déterminer le contour de l'objet cible sur chacune des données de sous-volume sur la base des instructions de l'utilisateur.

8. Système ultrasonique (100) selon la revendication 1, dans lequel la section de détermination de contour (125) est configurée pour détecter les points de contour de chacune des données de sous-volume et pour déterminer le contour de chacune des données de sous-volume sur la base des points de contour détectés.

9. Procédé pour produire des informations de volume d'un objet cible périodiquement mobile, consistant à :
a) transmettre/recevoir des signaux ultrasoniques de/vers un objet cible périodiquement mobile pour former ainsi des données ultrasoniques,
b) constituer des données de volume comprenant une pluralité de cadres basés sur les données ultrasoniques ;
c) former une pluralité d'images en tranches basées sur les données de volumes ;
d) fixer un point objet sur chacune des images en tranches sur la base des valeurs de brillance des pixels qui y sont inclus, pour former une courbe de points objets sur la base des points objets ;
e) calculer les points de croisement zéro dans le gradient de la courbe de points objets et détecter une période du point de croisement zéro détecté, comme étant une période mobile d'un objet cible, dans lequel la période mobile comprend une pluralité de périodes locales ;
f) effectuer une interpolation des données de volume pour avoir le même nombre de cadres dans chacune des périodes locales ;
g) reconstruire les données de volume de l'interpolation dans une pluralité de données de sous-volume, chaque donnée de sous-volume étant construite en utilisant les cadres dans une position identique dans chacune des périodes locales ;
h) déterminer le contour de l'objet cible dans chacune des données de sous-volume ; et
i) mesurer le volume de l'objet cible basé sur le contour afin de former ainsi l'information de volume.

10. Procédé selon la revendication 9, dans lequel l'étape d) consiste à :
déterminer le point objet sur chacune des images en tranches sur la base d'un centre géométrique des valeurs de la brillance de chaque tranche d'images ;
déterminer un axe principal basé sur les positions des points objets ; et
former la courbe des points objets basée sur la distance entre les points objets et l'axe principal.

11. Procédé selon la revendication 9, dans lequel l'étape e) consiste à :
calculer les gradients de la courbe des points objets ;
détecter les points de croisement zéro pour lesquels un signe du gradient change de positif en négatif ; et
déterminer la période de déplacement basée sur les intervalles entre les points de croisement zéro détectés.

12. Procédé selon la revendication 9, dans lequel l'étape h) consiste à :
déterminer le contour de l'objet cible sur chacune des données de sous-volume sur la base des instructions de l'utilisateur.

13. Procédé selon la revendication 9, dans lequel l'étape h) consiste à :
détecter les points de contour des données de sous-volume et pour déterminer le contour de chacune des données de sous-volume sur la base des points de contour détectés.
